# EUROPEAN PATENT APPLICATION

(11) **EP 3 892 337 A1**
(43) Date of publication of application: **13.10.2021**
(21) Application number: 19892247.8
(22) Date of filing: 04.12.2019
(51) Int. Cl.: A61Q 17/04, A61K 8/06, A61K 8/37, A61K 8/40, A61K 8/41, A61K 8/49, A61K 8/85

(54) **EMULSIFIED COSMETIC COMPOSITION**

(30) Priority: 07.12.2018 JP 2018229791
(71) Applicant: Shiseido Company, Ltd., Chuo-ku Tokyo 104-0061 (JP)
(72) Inventor: WATANABE Tsukasa, Tokyo 104-0061 (JP); YAMAGUCHI Kazuhiro, Tokyo 104-0061 (JP); WATANABE Yurika, Tokyo 104-0061 (JP)
(74) Representative: Ter Meer Steinmeister & Partner
(86) International application number: PCT/JP2019/047321
(87) International publication number: WO 2020/116487

(57) **Abstract**

An emulsion-type cosmetic composition contains from 3 to 50% by mass of an oil-soluble UV absorber, and from 0.2 to 20% by mass of a powder containing a polymer powder. An average IOB value of a monomer or monomers constituting the polymer powder is from 1 to 10.

## Description

### Related Application

The present application is based upon and claims the benefit of priority from Japanese Patent Application No. 2018-229791 filed on December 7, 2018, the entire contents of which are incorporated herein by reference.

### Technical Field

The present disclosure relates to an emulsion-type cosmetic composition. For example, the present disclosure relates to an emulsion-type cosmetic composition containing a UV absorber.

### Background Art

Patent Literatures 1 and 2 disclose oil-in-water sun-block cosmetic products containing a UV absorber.

Patent Literature 1 discloses an oil-in-water emulsion-type sun-block cosmetic product containing (a) an oil-soluble UV absorber, (b) a water-soluble thickener, (c) a water-soluble UV absorber, and (d) at least one type of hydrophilic nonionic surfactant selected from PEG glyceryl fatty acid ester-based surfactants, hydrogenated castor oil-based surfactants, and PEG-PPG alkyl ether-based surfactants.

Patent Literature 2 attempts to improve the effect of the UV absorber in an oil-in-water sun-block cosmetic product. Patent Literature 2 discloses an oil-in-water sun-block cosmetic product in which the sun protection factor (SPF) is improved by combining phenylbenzimidazole sulfonic acid, which is a water-soluble UV absorber, and N,N,N',N'-tetrakis(2-hydroxypropyl)ethylenediamine, which is a neutralizer of phenylbenzimidazole sulfonic acid.

### Citation List

### Patent Literature

Patent Literature 1: Japanese Unexamined Patent Publication No. 2008-162930A
Patent Literature 2: Japanese Unexamined Patent Publication No. 2011-111444A

### Summary of Invention

### Technical Problem

The following analysis can be made from the perspective of the present disclosure.

Cosmetics containing oil-soluble UV absorbers often take the form of an emulsion. Unfortunately, in emulsion-type cosmetics containing oil-soluble UV absorbers as disclosed in Patent Literature 1, the oil-soluble UV absorbers may not be able to exert their UV protection effects sufficiently.

The method for improving UV protection effects disclosed in Patent Literature 2 is useful for specific water-soluble UV absorbers, but is, unfortunately, not a versatile method. Also, the method disclosed in Patent Literature 2 is not applicable to oil-soluble UV absorbers.

Thus, there is a demand for an emulsion-type cosmetic composition that can enable an oil-soluble UV absorber to exert its effect more efficiently.

### Solution to Problem

According to a first aspect of the present disclosure, an emulsion-type cosmetic composition is provided, the composition comprising from 3 to 50% by mass of an oil-soluble UV absorber, and from 0.2 to 20% by mass of a powder containing a polymer powder. An average IOB value of a monomer or monomers constituting the polymer powder is from 1 to 10.

### Advantageous Effects of Invention

The emulsion-type cosmetic composition of the present disclosure can enable an oil-soluble UV absorber to exert its UV protection effect sufficiently.

### Description of Embodiments

Preferred modes according to the aforementioned aspects will be described below.

According to a prefered mode of the above first aspect, the polymer powder has an average particle size of from 0.5 to 20 µm.

According to a prefered mode of the above first aspect, the polymer powder has a squalane oil absorption value of 45 mL/100 g or greater.

According to a prefered mode of the above first aspect, the polymer powder contains a polybutylene succinate powder and/or a polylactic acid powder.

According to a prefered mode of the above first aspect, wherein the oil-soluble UV absorber contains at least one selected from the group consisting of homosalate, ethylhexyl salicylate, octocrylene, bis-ethylhexyloxyphenol methoxyphenyl triazine, and diethylamino hydroxybenzoyl hexyl benzoate.

In the following description, POE is an abbreviation of polyoxyethylene, and POP is an abbreviation of polyoxypropylene. The number in parentheses after POE or POP indicates the average number of moles of POE groups or POP groups added in the compound in question.

In the present disclosure, "substantial amount" refers to an amount capable of bringing about effects due to addition of the compound in question.

An emulsion-type cosmetic composition according to a first embodiment of the present disclosure will be described. The emulsion-type cosmetic composition of the present disclosure may be an oil-in-water-type composition or a water-in-oil-type composition.

The emulsion-type cosmetic composition according to the first embodiment contains: (A) an oil-soluble UV absorber; and (B) a powder. Component (B) can improve the UV protection effect of component (A).

### (A) Oil-Soluble UV Absorber:

Examples of oil-soluble UV absorbers may include: benzoic acid-based UV absorbers (e.g., para-aminobenzoic acid (abbreviated as PABA hereinbelow), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, diethylamino hydroxybenzoyl hexyl benzoate, etc.); anthranilic acid-based UV absorbers (e.g., homomenthyl-N-acetylanthranilate, etc.); salicylic acid-based UV absorbers (e.g., ethylhexyl salicylate, amyl salicylate, menthyl salicylate, homomenthyl salicylate, octyl salicylate, phenyl salicylate, benzyl salicylate, p-isopropanol phenyl salicylate, homosalate, etc.); cinnamic acid-based UV absorbers (e.g., octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, ethylhexyl methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, glyceryl mono-2-ethylhexanoyl-di-para-methoxycinnamate, etc.); 3-(4'-methylbenzylidene)-d,l-camphor, 3-benzylidene-d,1-camphor; 2-phenyl-5-methylbenzoxazole; 2,2'-hydroxy-5-methylphenyl benzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenyl acrylate (octocrylene); 2,4-bis-{[4-(2-ethylhexyloxy)-2-hydroxy]-phenyl}-6-(4-methoxyphenyl)-(1,3,5)-tri azine; and benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.).

From among the aforementioned UV absorbers, from the viewpoint of UV protection effects, it is possible to select, for example, at least one from the group consisting of homosalate, ethylhexyl salicylate, octocrylene, bis-ethylhexyloxyphenol methoxyphenyl triazine, and diethylamino hydroxybenzoyl hexyl benzoate.

The content by percentage of the oil-soluble UV absorber in the emulsion-type cosmetic composition can be set as appropriate depending on the purpose. The content by percentage of the oil-soluble UV absorber with respect to the mass of the composition may be, for example, 1% by mass or greater, 2% by mass or greater, 3% by mass or greater, or 5% by mass or greater. The content by percentage of the oil-soluble UV absorber with respect to the mass of the composition may be, for example, 50% by mass or less, 40% by mass or less, 30% by mass or less, 25% by mass or less, 20% by mass or less, 15% by mass or less, 12% by mass or less, 10% by mass or less, or 8% by mass or less.

### (B) Powder for Improving UV Protection Effect:

Component (B) may be a polymer powder. The polymer powder may be, for example, an organic polymer powder. The average IOB value of the polymer may be 1 or greater, 1.5 or greater, 1.8 or greater, or 2 or greater. The average IOB value of the polymer may be 10 or less, 8 or less, 7 or less, 6 or less, 5 or less, or 4 or less. By setting the average IOB value within this range, the UV protection effect of the oil-soluble UV absorber can be improved.

From the viewpoint of usability, component (B) is preferably an organic polymer. For the organic polymer powder, it is possible to use, for example, a polybutylene succinate powder and/or a polylactic acid powder. Polybutylene succinate and polylactic acid are generally known as biodegradable resins.

Herein, "IOB value" refers to the ratio (inorganic value (IV)/organic value (OV)) of the inorganic value (IV) to the organic value (OV) in the organic conceptual diagram. For details on the IOB value, see the description in Japanese Unexamined Patent Publication No. 2010-100553A which is incorporated herein by reference.

The average IOB value of the polymer is the average value of IOB values of monomers constituting the polymer. For example, the average IOB value of polybutylene succinate is the average value of the IOB value of succinic acid and the IOB value of 1,4-hexanediol. Further, for example, the average IOB value of polylactic acid is the IOB value of lactic acid.

The squalane oil absorption value of component (B) may be, for example, 45 mL/100 g or greater, 48 mL/100 g or greater, 50 mL/100 g or greater, or 55 mL/100 g or greater. By setting the squalane oil absorption value to 45 mL/100 g or greater, the UV protection effect of the oil-soluble UV absorber can be further improved. The squalane oil absorption value of component (B) may be, for example, 85 mL/100 g or less, 80 mL/100 g or less, 75 mL/100 g or less, 70 mL/100 g or less, 65 mL/100 g or less, or 60 mL/100 g or less.

The squalane oil absorption value of component (B) can be measured according to JIS K 5101-13-1.

The average particle size D50 of component (B) may be, for example, 0.5 µm or greater, 1 µm or greater, 2 µm or greater, 3 µm or greater, or 4 µm or greater. Particularly, by setting the average particle size to 2 µm or greater, it is possible to suppress squeakiness and improve the feel upon use of the cosmetic composition. The average particle size D50 of component (B) may be, for example, 20 µm or less, 15 µm or less, or 10 µm or less. By setting the average particle size to 20 µm or less, the UV protection effect of the oil-soluble UV absorber can be further improved.

The shape of the particles in component (B) may be, for example, spherical.

The content by percentage of component (B) with respect to the mass of the composition is preferably 0.2% by mass or greater, more preferably 0.5% by mass or greater, even more preferably 0.8% by mass or greater.

The content by percentage of component (B) with respect to the mass of the composition may be, for example, 1% by mass or greater, 2% by mass or greater, 3% by mass or greater, 5% by mass or greater, or 10% by mass or greater. The content by percentage of component (B) with respect to the mass of the composition may be, for example, 20% by mass or less, 15% by mass or less, 10% by mass or less, or 5% by mass or less.

### (B') Other Powders:

Other than component (B), the emulsion-type cosmetic composition according to the first embodiment may contain (B') other powders.

Component (B') may include a UV scattering agent. The content by percentage of the UV scattering agent with respect to the mass of the composition may be, for example, 2% by mass or greater, 5% by mass or greater, or 10% by mass or greater. The content by percentage of the UV scattering agent with respect to the mass of the composition may be, for example, 20% by mass or less, 15% by mass or less, or 10% by mass or less. The content by percentage of the UV scattering agent can be set as appropriate depending on the use.

The term "powder body" used herein is synonymous to "powder." Component (B') is not particularly limited, so long as it can be used for general purposes, such as cosmetic uses, etc. Examples of Component (B') may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No. 104, Red No. 106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc); wax powder (such as carnauba wax powder, etc.); starch powder (such as cornstarch powder, rice starch powder, etc) and the like.

In the emulsion-type cosmetic composition according to the first embodiment, the content by percentage of the silicone powder with respect to the mass of the composition may be 2% by mass or less, 1% by mass or less, or 0.5% by mass or less. The emulsion-type cosmetic composition may contain substantially no silicone powder. By containing substantially no silicone powder in the emulsion-type cosmetic composition, it is possible to achieve an even smoother feel upon use.

The content by percentage of component (B') with respect to the mass of the composition may be, for example, 1% by mass or greater, 2% by mass or greater, 3% by mass or greater, 5% by mass or greater, or 10% by mass or greater. The content by percentage of component (B') with respect to the mass of the composition may be, for example, 20% by mass or less, 15% by mass or less, 10% by mass or less, or 5% by mass or less.

### (C) Oily Component:

The emulsion-type cosmetic composition of the present disclosure may further contain an oily component. It is preferred that at least a portion of the oily component is capable of dissolving the oil-soluble UV absorber. It will suffice if the oily component is in liquid form as a whole, and may contain solid components. Examples of usable oily components may include liquid oils, solid fats, waxes, hydrocarbons, higher fatty acids, higher alcohols, synthetic ester oils, and silicone oils.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

It is preferred that the oily component contains a volatile oily component. The volatile oily component may be capable of dissolving the oil-soluble UV absorber, or may have difficulty in dissolving the oil-soluble UV absorber. Examples of the volatile oily component may include hydrocarbons and silicone oils. Examples of the volatile oily component may include linear silicone oils (e.g., volatile dimethicone), volatile cyclic silicone oils (e.g., volatile cyclomethicone), isododecane, and isohexadecane.

The percentage of the volatile oily component in the oily component (including the oil-soluble UV absorber) may be, for example, 5% by mass or greater, 10% by mass or greater, 20% by mass or greater, or 30% by mass or greater, with respect to the mass of the oily component. The percentage of the volatile oily component in the oily component (including the oil-soluble UV absorber) may be, for example, 60% by mass or less, 50% by mass or less, or 40% by mass or less, with respect to the mass of the oily component.

The content by percentage of the volatile oily component with respect to the mass of the composition may be, for example, 1% by mass or greater, 2% by mass or greater, 3% by mass or greater, or 5% by mass or greater. The content by percentage of the volatile oily component with respect to the mass of the composition may be, for example, 50% by mass or less, 40% by mass or less, 30% by mass or less, 20% by mass or less, or 10% by mass or less. The content by percentage of the volatile oily component can be set as appropriate depending on the use.

The content by percentage of the oily component (including the oil-soluble UV absorber) with respect to the mass of the composition may be, for example, 15% by mass or greater, 20% by mass or greater, or 30% by mass or greater. The content by percentage of the oily component (including the oil-soluble UV absorber) with respect to the mass of the composition may be, for example, 60% by mass or less, 50% by mass or less, or 40% by mass or less. The content by percentage of the oily component can be set as appropriate depending on the use.

### (D) Aqueous Solvent:

The emulsion-type cosmetic composition of the present disclosure may further contain an aqueous solvent. It is preferred that the aqueous solvent contains water. Examples of water usable herein may include water used for cosmetics, quasi-pharmaceutical products, or the like, and usable examples may include purified water, ion-exchanged water, tap water, etc.

The aqueous solvent may further include a water-soluble alcohol. Examples of the water-soluble alcohol may include at least one selected from, for example, lower alcohols, polyols, polyol polymers, divalent alcohol alkyl ethers, divalent alcohol alkyl ethers, divalent alcohol ether esters, glycerin monoalkyl ethers, sugar alcohols, monosaccharides, oligosaccharides, polysaccharides, and derivatives thereof.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

The content by percentage of the aqueous solvent with respect to the mass of the composition may be, for example, 10% by mass or greater, 20% by mass or greater, or 30% by mass or greater. The content by percentage of the aqueous solvent with respect to the mass of the composition may be, for example, 60% by mass or less, 50% by mass or less, or 40% by mass or less. The content by percentage of the aqueous solvent can be set as appropriate depending on the use.

The ratio between the oily component and the aqueous component can be set as appropriate.

### (E) Surfactant:

The emulsion-type cosmetic composition of the present disclosure may further contain a surfactant. Examples of surfactants may include the following surfactants.

### Anionic Surfactant:

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

### Cationic Surfactant:

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, amino acid-based cationic surfactant (such as ethyl L-cocoyl arginine DL-pyrrolidonecarboxylic acid salt) and the like.

### Amphoteric Surfactant:

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

### Hydrophilic Nonionic Surfactant:

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

### Lipopholic Nonionic Surfactant:

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

The content by percentage of the surfactant with respect to the mass of the composition is preferably 0.1% by mass or greater, more preferably 0.5% by mass or greater. If the content of the surfactant is less than 0.1% by mass, an emulsified composition may not be obtained. The content by percentage of the surfactant with respect to the mass of the composition is preferably 10% by mass or less. If the content by percentage of the surfactant exceeds 10% by mass, stimulation to the skin may become too strong.

### (F) Others:

If necessary, the emulsion-type cosmetic composition of the present disclosure may contain other components as appropriate, such as water-soluble polymers, thickeners, moisturizers, film-forming agents, water-soluble UV absorbers, metal ion sequestering agents, amino acids, organic amines, polymer emulsions, pH adjusters, skin nutrients, vitamins, antioxidants, antioxidant aids, perfumes, etc., in amounts that do not inhibit the effects of the present disclosure.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat), glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of other thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride, taurate-based synthetic polymers, and acrylate-based synthetic polymers.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of water-soluble UV absorbers may include: benzophenone-based UV absorbers (e.g. 2-hydroxy-4-methoxybenzophenone-5-sulfate, etc.); benzylidene camphor-based UV absorbers (e.g. benzylidene camphor sulfonic acid, terephthalylidene dicamphor sulfonic acid, etc.); phenylbenzimidazole-based UV absorbers (e.g. phenylbenzimidazole sulfonic acid, etc.), and the like.

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure may further contain, as appropriate: caffeine, tannin, verapamil, tranexamic acid and derivatives thereof; various herbal medicine extracts such as licorice, pseudocydonia sinensis, pyrola japonica, etc.; drugs such as tocopherol acetate, glycyrrhetinic acid, glycyrrhizinic acid and derivatives or salts thereof; skin whitening agents such as vitamin C, magnesium ascorbyl phosphate, ascorbic acid glucoside, arbutin, kojic acid, etc.; amino acids such as arginine, lysine, etc., and derivatives thereof.

With the emulsion-type cosmetic composition of the present disclosure, the blended oil-soluble UV absorber can be made to exert its actions more efficiently. Thus, the UV protection effect can be improved.

With the emulsion-type cosmetic composition of the present disclosure, a sun-block cosmetic product containing an aqueous phase can be prepared. The presence of the aqueous phase can offer the user a fresh and moist feel upon use.

### Production Method:

A method for producing the emulsion-type cosmetic composition of the present disclosure will be described. The emulsion-type cosmetic composition of the present disclosure can be prepared by any generally known method, without being limited to a specific method. For example, the emulsion-type cosmetic composition can be prepared by mixing each of the aforementioned components.

There may be cases where it is difficult, or utterly impractical, to directly define the phase structure etc. of the emulsion-type cosmetic composition of the present disclosure based on the compositional makeup thereof. In such circumstances, it should be permissible to define the emulsion-type cosmetic composition of the present disclosure according to methods for producing the same.

### Method of Use:

A method for using the emulsion-type cosmetic composition of the present disclosure and a method for improving UV protection effects will be described. First, an emulsion-type cosmetic composition containing an oil-soluble UV absorber and a polymer powder is prepared. Next, the emulsion-type cosmetic composition is applied to the skin. As for the oil-soluble UV absorber, the polymer powder, and the emulsion-type cosmetic composition, the above description is incorporated herein by reference.

### Examples

The emulsion-type cosmetic composition of the present disclosure will be described by way of examples. Note, however, that the emulsion-type cosmetic composition of the present disclosure is not limited to the following examples. The following Examples describe examples wherein the emulsion-type cosmetic compositions according to the respective Test Examples are employed as sun-block cosmetic products, but the composition of the present disclosure is not limited to sun-block cosmetic products. The content by percentage of each of the components shown in the Tables is in terms of percent by mass (mass%).

The average IOB value and the squalane oil absorption value of each powder, i.e., component (B), blended to the respective compositions in the Test Examples are shown in Table 1. The average IOB value is the average value of IOB value(s) of monomer(s) before polymerization. The squalane oil absorption value was measured according to JIS K 5101-13-1. The shape of particles in each powder shown in Table 1 was spherical.

**[Table 1]**

| Powder | Average IOB value | Squalane oil absorptio n value (mL/100g) |
|---|---|---|
| Polybutylene succinate A | 2.2 | 53 |
| Polylactic acid A *¹ | 3.3 | 75 |
| Polylactic acid B *² | | 53 |
| *1: MAKIBEADS ECO D-1 (Daito Kasei Kogyo Co., Ltd.) | | |
| *2: MAKIBEADS ECO D-5 (Daito Kasei Kogyo Co., Ltd.) | | |

To a polymethyl methacrylate (PMMA) plate (SPF MASTER-PA01) serving as artificial skin, 2 mg/cm² of each composition was applied at room temperature (25°C) with the fingers for 60 seconds, and each composition was dried for 15 minutes, to form respective coating films of the respective sun-block cosmetic products. For each coating film, the absorbance within the range from 280 to 400 nm was measured using an absorptiometer (U-3500 from Hitachi, Ltd.), to calculate the integrated value of the absorbance. Compositions containing the same oil-soluble UV absorber(s) but not including polybutylene succinate powder or polylactic acid powder as component (B) were prepared as respective Control Examples. The following equation was employed to calculate the rate of increase in the integrated value of the absorbance for each composition according to the respective Test Example containing polybutylene succinate powder or polylactic acid powder. The rate of increase in the integrated value of the absorbance may serve as an index of the UV protection effect based on the respective UV absorbers.

Rate of increase in integrated value of absorbance (%) = Integrated value of absorbance of composition containing component (B)/Integrated value of absorbance of composition not containing component (B) × 100.

### Test Examples 1 to 6:

Water-in-oil cosmetic compositions having the respective compositional makeup shown in Tables 2 and 3 were prepared. The water-in-oil cosmetic compositions according to the Test Examples are sun-block cosmetic products. The integrated value of absorbance and the rate of increase thereof are shown in Tables 2 and 3. The rate of increase in the integrated value of absorbance in each of Test Examples 1 to 3, 5 and 6 is the rate of increase with respect to the integrated value of absorbance in Test Example 4.

Test Examples 5 and 6 did not contain component (A), i.e., an oil-soluble UV absorber. A comparison between Test Example 5 which contained component (B) and Test Example 6 which did not contain component (B) showed no significant difference in the integrated value of absorbance. This suggests that, even if component (B) is added to a composition containing no component (A), it is not possible to improve the UV protection effect of the oil-soluble UV absorber.

In contrast, Test Examples 1 to 4 contained component (A). A comparison between Test Examples 1 to 3 which contained component (B) and Test Example 4 which did not contain component (B) showed that the integrated value of absorbance increased by adding component (B). Stated differently, the combined use of component (A) and component (B) showed an increase in the integrated value of absorbance. This suggests that component (B) acts on component (A), thereby improving the UV protection effect of the oil-soluble UV absorber.

Test Example 1 used a polybutylene succinate powder as component (B). Test Examples 2 and 3 used a polylactic acid powder. Both the polybutylene succinate powder and the polylactic acid powder showed an increase in the integrated value of absorbance. Stated differently, the results show that both the polybutylene succinate powder and the polylactic acid powder act to improve the UV protection effect of the oil-soluble UV absorber in the emulsion-type compositions.

Test Examples 1 to 3 suggest that, when the average IOB value of component (B) is 1 or greater, the UV protection effect can be improved. It is thought that the average IOB value of component (B) may be 10 or less.

Test Examples 1 to 3 also suggest that the squalane oil absorption value of component (B) may be 45 mL/100 g or greater. The results also suggest that the average particle size of component (B) may be 85 mL/100 g or less.

**[Table 2]**

| Test Example | 1 | 2 | 3 |
|---|---|---|---|
| (A) Ethylhexyl methoxycinnamate | 7 | 7 | 7 |
| (A) Octocrylene | 3 | 3 | 3 |
| (A) Bis-ethylhexyloxyphenol methoxyphenyl triazine | 1 | 1 | 1 |
| (A) Diethylamino hydroxybenzoyl hexyl benzoate | 1 | 1 | 1 |
| (B) Polybutylene succinate A | 5 | - | - |
| (B) Polylactic acid A *¹ | - | 5 | - |
| (B) Polylactic acid B *² | - | - | 5 |
| (B') Hydrophobized titanium oxide | 1 | 1 | 1 |
| (B') Hydrophobized zinc oxide | 10 | 10 | 10 |
| (C) Cyclopentasiloxane | 23 | 23 | 23 |
| (C) Isododecane | 8 | 8 | 8 |
| (C) Dimethicone | 2 | 2 | 2 |
| (C) Isostearic acid | 1 | 1 | 1 |
| (C) Isopropyl myristate | 6 | 6 | 6 |
| (C) PPG-17 | 1 | 1 | 1 |
| (D) Ethanol | 10 | 10 | 10 |
| (D) Glycerine | 1 | 1 | 1 |
| (D) Ion-exchanged water | Balanc e | Balanc e | Balanc e |
| (E) Sorbitan sesquiisostearate | 1 | 1 | 1 |
| (E) PEG-9 polydimethylsiloxyethyl dimethicone | 1 | 1 | 1 |
| (F) Disteardimonium hectorite | 0.5 | 0.5 | 0.5 |
| Total | 100 | 100 | 100 |
| Integrated value of absorbance | 139 | 144 | 154 |
| Rate of increase in integrated value of absorbance (%) | 108 | 112 | 120 |

**[Table 3]**

| Test Example | 4 | 5 | 6 |
|---|---|---|---|
| (A) Ethylhexyl methoxycinnamate | 7 | - | - |
| (A) Octocrylene | 3 | - | - |
| (A) Bis-ethylhexyloxyphenol methoxyphenyl triazine | 1 | - | - |
| (A) Diethylamino hydroxybenzoyl hexyl benzoate | 1 | - | - |
| (B) Polybutylene succinate A | - | 5 | - |
| (B) Polylactic acid A *¹ | - | - | - |
| (B) Polylactic acid B *² | - | - | - |
| (B') Hydrophobized titanium oxide | 1 | 1 | 1 |
| (B') Hydrophobized zinc oxide | 10 | 10 | 10 |
| (C) Cyclopentasiloxane | 23 | 23 | 23 |
| (C) Isododecane | 8 | 8 | 8 |
| (C) Dimethicone | 2 | 2 | 2 |
| (C) Isostearic acid | 1 | 1 | 1 |
| (C) Isopropyl myristate | 6 | 6 | 6 |
| (C) PPG-17 | 1 | 1 | 1 |
| (D) Ethanol | 10 | 10 | 10 |
| (D) Glycerine | 1 | 1 | 1 |
| (D) Ion-exchanged water | Balanc e | Balanc e | Balanc e |
| (E) Sorbitan sesquiisostearate | 1 | 1 | 1 |
| (E) PEG-9 polydimethylsiloxyethyl dimethicone | 1 | 1 | 1 |
| (F) Disteardimonium hectorite | 0.5 | 0.5 | 0.5 |
| Total | 100 | 100 | 100 |
| Integrated value of absorbance | 129 | 67 | 67 |
| Rate of increase in integrated value of absorbance (%) | 100 | 52 | 52 |

### Test Examples 7 to 14:

As in Test Examples 1 to 6, water-in-oil cosmetic compositions having the respective compositional makeup shown in Tables 4 and 5 were prepared. The water-in-oil cosmetic compositions according to the Test Examples are sun-block cosmetic products. The integrated value of absorbance and the rate of increase thereof are shown in Tables 4 and 5. The rate of increase in the integrated value of absorbance in each of Test Examples 7 to 9 is the rate of increase with respect to the integrated value of absorbance in Test Example 10. The rate of increase in the integrated value of absorbance in each of Test Examples 11 to 13 is the rate of increase with respect to the integrated value of absorbance in Test Example 14.

Test Examples 7 to 10 differ from Test Examples 11 to 14 in terms of whether or not the compositions contain a mineral-based powder, an acrylic resin-based powder, and a silicone-based powder. Despite this difference, both Test Examples 7 to 10 and Test Examples 11 to 14 showed an increase in the integrated value of absorbance caused by the addition of component (B). This suggests that the addition of a mineral-based powder, an acrylic resin-based powder, and a silicone-based powder does not affect the improvement of UV protection effect brought about by the polybutylene succinate powder and/or the polylactic acid powder.

**[Table 4]**

| Test Example | 7 | 8 | 9 | 10 |
|---|---|---|---|---|
| (A) Ethylhexyl methoxycinnamate | 7 | | 7 | 7 |
| (A) Octocrylene | 3 | 3 | 3 | 3 |
| (A) Bis-ethylhexyloxyphenol methoxypheny l triazine | 1 | 1 | 1 | 1 |
| (A) Diethylamino hydroxybenzoyl hexyl be nzoate | 1 | 1 | 1 | 1 |
| (A) Terephthalylidene dicamphor sulfonic acid | 2 | 2 | 2 | 2 |
| (B) Polybutylene succinate A | 5 | - | - | - |
| (B) Polylactic acid A *¹ | - | 5 | - | - |
| (B) Polylactic acid B *² | - | - | 5 | |
| (B') Hydrophobized titanium oxide | 2 | 2 | 2 | 2 |
| (B') Hydrophobized zinc oxide | 10 | 10 | 10 | 10 |
| (C) Cyclopentasiloxane | 14 | 9 | 9 | 9 |
| (C) Isododecane | 3 | 3 | 3 | 3 |
| (C) Dimethicone | 20 | 20 | 20 | 20 |
| (C) Isostearic acid | 0.5 | 0.5 | 0.5 | 0.5 |
| (C) Isopropyl myristate | 5 | 5 | 5 | 5 |
| (C) Diisopropyl sebacate | 2 | 2 | 2 | 2 |
| (C) PPG-17 | 1 | 1 | 1 | 1 |
| (C) Stearic acid | 1 | 1 | 1 | 1 |
| (C) Dextrin palmitate | 1 | 1 | 1 | 1 |
| (D) Ethanol | 9 | 9 | 9 | 9 |
| (D) Glycerin | 1 | 1 | 1 | 1 |
| (D) Ion-exchanged water | Balance | Balance | Balance | Balance |
| (E) PEG-9 polydimethylsiloxyethyl dimethi cone | 1.5 | 1.5 | 1.5 | 1.5 |
| (E) Distearyldimonium chloride | 0.3 | 0.3 | 0.3 | 0.3 |
| (F) Polyoxyethylene (14) polyoxypropylene | 1 | 1 | 1 | 1 |
| (7) dimethyl ether | | | | |
| (F) Disteardimonium hectorite | 0.3 | 0.3 | 0.3 | 0.3 |
| (F) Triethanol amine | 1.3 | 1.3 | 1.3 | 1.3 |
| (F) Stabilizing agent | Appropr iate am ount | Appropr iate am ount | Appropr iate am ount | Appropr iate am ount |
| (F) Perfume | Appropr iate am ount | Appropr iate am ount | Appropr iate am ount | Appropr iate am ount |
| Total | 100 | 100 | 100 | 100 |
| Integrated value of absorbance | 144 | 166 | 176 | 131 |
| Rate of increase in integrated value of ab sorbance (%) | 110 | 127 | 135 | 100 |

**[Table 5]**

| Test Example | 11 | 12 | 13 | 14 |
|---|---|---|---|---|
| (A) Ethylhexyl methoxycinnamate | 7 | 7 | 7 | 7 |
| (A) Octocrylene | 3 | 3 | 3 | 3 |
| (A) Bis-ethylhexyloxyphenol methoxypheny l triazine | 1 | 1 | 1 | 1 |
| (A) Diethylamino hydroxybenzoyl hexyl be nzoate | 1 | 1 | 1 | 1 |
| (A) Terephthalylidene dicamphor sulfonic acid | 2 | 2 | 2 | 2 |
| (B) Polybutylene succinate A | 5 | - | - | - |
| (B) Polylactic acid A *¹ | - | 5 | - | - |
| (B) Polylactic acid B *² | - | - | 5 | - |
| (B') Talc | 4 | 4 | 4 | 4 |
| (B') Polymethyl methacrylate | 3 | 3 | 3 | 3 |
| (B') (Vinyl dimethicone/methicone silsesqui oxane) crosspolymer | 1 | 1 | 1 | 1 |
| (B') Hydrophobized titanium oxide | 2 | 2 | 2 | 2 |
| (B') Hydrophobized zinc oxide | 10 | 10 | 10 | 10 |
| (C) Cyclopentasiloxane | 13 | 8 | 8 | 8 |
| (C) Isododecane | 3 | 3 | 3 | 3 |
| (C) Dimethicone | 19 | 19 | 19 | 19 |
| (C) Isostearic acid | 0.5 | 0.5 | 0.5 | 0.5 |
| (C) Isopropyl myristate | 5 | 5 | 5 | 5 |
| (C) Diisopropyl sebacate | 2 | 2 | 2 | 2 |
| (C) PPG-17 | 1 | 1 | 1 | 1 |
| (C) Stearic acid | 1 | 1 | 1 | 1 |
| (C) Dextrin palmitate | 1 | 1 | 1 | 1 |
| (D) Ethanol | 9 | 9 | 9 | 9 |
| (D) Glycerin | 1 | 1 | 1 | 1 |
| (D) Ion-exchanged water | Balance | Balance | Balance | Balance |
| (E) PEG-9 polydimethylsiloxyethyl dimethicone | 1.5 | 1.5 | 1.5 | 1.5 |
| (E) Distearyldimonium chloride | 0.3 | 0.3 | 0.3 | 0.3 |
| (F) Polyoxyethylene (14) polyoxypropylene (7) dimethyl ether | 1 | 1 | 1 | 1 |
| (F) Disteardimonium hectorite | 0.3 | 0.3 | 0.3 | 0.3 |
| (F) Triethanol amine | 1.3 | 1.3 | 1.3 | 1.3 |
| (F) Stabilizing agent | Appropriate amount | Appropriate amount | Appropriate amount | Appropriate amount |
| (F) Perfume | Appropriate amount | Appropriate amount | Appropriate amount | Appropriate amount |
| Total | 100 | 100 | 100 | 100 |
| Integrated value of absorbance | 161 | 175 | 191 | 156 |
| Rate of increase in integrated value of absorbance (%) | 104 | 113 | 123 | 100 |

### Test Examples 15 to 23:

As in Test Examples 1 to 6, water-in-oil cosmetic compositions having the respective compositional makeup shown in Tables 7 and 8 were prepared. The water-in-oil cosmetic compositions according to the Test Examples are sun-block cosmetic products. The integrated value of absorbance and the rate of increase thereof are shown in Tables 7 and 8. The rate of increase in the integrated value of absorbance in each of Test Examples 16 to 23 is the rate of increase with respect to the integrated value of absorbance in Test Example 15.

The powder, i.e., component (B), blended to the respective compositions in Test Examples 16 to 18 was lactic acid A shown in Table 1. The powder, i.e., component (B), blended to the respective compositions in Test Examples 19 to 21 was polybutylene succinate A shown in Table 1. The average IOB value and the squalane oil absorption value of polymethyl methacrylate and the polymethylsilsesquioxane powder blended to the respective compositions in Test Examples 22 and 23 are shown in Table 6. The average IOB value is the average value of IOB value(s) of monomer(s) before polymerization. The squalane oil absorption value was measured according to JIS K 5101-13-1. The shape of particles in each powder shown in Table 6 was spherical.

**[Table 6]**

| Powder | Average particl e size D50 (µm) | Average IOB value | Squalane oil abs orption value (mL/100g) |
|---|---|---|---|
| Polymethyl methacrylate *³ | 7.3 | 0.775 | 43 |
| Polymethylsilsesquioxane *⁴ | 6 | 0.47 | - |
| *3: Ganzpearl (registered trademark) GMX-0810 (Aica Kogyo Co., Ltd.) | | | |
| *4: Tospearl (registered trademark) 2000B (Momentive Performance Materials Japan ) | | | |

Test Example 16, containing 0.1% by mass of polylactic acid as component (B), and Test Example 19, containing 0.1% by mass of polybutylene succinate, did not show an increase in the integrated value of absorbance. In contrast, Test Example 16, containing 0.5% by mass of polylactic acid, showed an increase in the integrated value of absorbance. This suggests that the content of component (B) is preferably 0.2% by mass or greater, more preferably 0.5% by mass or greater.

Test Example 18, containing 10% by mass of polylactic acid as component (B), and Test Examples 20 and 21, respectively containing 10% by mass and 15% by mass of polybutylene succinate, showed an increase in the integrated value of absorbance. This suggests that the content of component (B) may at least be 20% by mass or less.

Test Examples 22 and 23, respectively containing polymethyl methacrylate whose monomer has an average IOB value of approximately 0.8 and polymethylsilsesquioxane having an average IOB value of approximately 0.5, showed a decrease in the integrated value of absorbance. This suggests that the average IOB value is preferably 1 or greater.

**[Table 7]**

| Test Example | 15 | 16 | 17 | 18 |
|---|---|---|---|---|
| (A) Ethylhexyl methoxycinnamate | 7.5 | 7.5 | 7.5 | 7.5 |
| (A) Octocrylene | 3 | 3 | 3 | 3 |
| (A) Bis-ethylhexyloxyphenol methoxyphenyl triazine | 0.5 | 0.5 | 0.5 | 0.5 |
| (A) Diethylamino hydroxybenzoyl hexyl ben zoate | 1.5 | 1.5 | 1.5 | 1.5 |
| (B) Polylactic acid A *¹ | - | 0.1 | 0.5 | 10 |
| (B) Polybutylene succinate A | - | - | - | - |
| Polymethyl methacrylate *³ | - | - | - | - |
| Polymethylsilsesquioxane *⁴ | - | - | - | - |
| (B') Hydrophobized titanium oxide | 2 | 2 | 2 | 2 |
| (B') Hydrophobized zinc oxide | 9 | 9 | 9 | 9 |
| (C) Cyclopentasiloxane | 18 | 18 | 18 | 18 |
| (C) Isododecane | 8 | 8 | 8 | 8 |
| (C) Dimethicone | 2 | 2 | 2 | 2 |
| (C) Isostearic acid | 0.25 | 0.25 | 0.25 | 0.25 |
| (C) Isopropyl myristate | 5 | 5 | 5 | 5 |
| (C) Diisopropyl sebacate | 1 | 1 | 1 | 1 |
| (C) PPG-17 | 1 | 1 | 1 | 1 |
| (D) Ethanol | 10 | 10 | 10 | 10 |
| (D) Glycerin | 1 | 1 | 1 | 1 |
| (D) Ion-exchanged water | Balance | Balance | Balance | Balance |
| (E) Sorbitan sesquiisostearate | 0.5 | 0.5 | 0.5 | 0.5 |
| (E) PEG-9 polydimethylsiloxyethyl dimethic one | 0.6 | 0.6 | 0.6 | 0.6 |
| (F) Disteardimonium hectorite | 0.5 | 0.5 | 0.5 | 0.5 |
| (F) Tocopherol | 0.05 | 0.05 | 0.05 | 0.05 |
| (F) Dibutylhydroxytoluene | 0.05 | 0.05 | 0.05 | 0.05 |
| (F) EDTA-3Na | 0.1 | 0.1 | 0.1 | 0.1 |
| Total | 100 | 100 | 100 | 100 |
| Integrated value of absorbance | 51.9 | 51.0 | 58.5 | 53.8 |
| Rate of increase in integrated value of abso rbance (%) | 100 | 98 | 113 | 146 |

**[Table 8]**

| Test Example | 19 | 20 | 21 | 22 | 23 |
|---|---|---|---|---|---|
| (A) Ethylhexyl methoxycinnamate | 7.5 | 7.5 | 7.5 | 7.5 | 7.5 |
| (A) Octocrylene | 3 | 3 | 3 | 3 | 3 |
| (A) Bis-ethylhexyloxyphenol methox yphenyl triazine | 0.5 | 0.5 | 0.5 | 0.5 | 0.5 |
| (A) Diethylamino hydroxybenzoyl h exyl benzoate | 1.5 | 1.5 | 1.5 | 1.5 | 1.5 |
| (B) Polylactic acid A *¹ | - | - | - | - | - |
| (B) Polybutylene succinate A | 0.1 | 10 | 15 | - | - |
| Polymethyl methacrylate *³ | - | - | - | 5 | - |
| Polymethylsilsesquioxane *⁴ | - | - | - | | 5 |
| (B') Hydrophobized titanium oxide | 2 | 2 | 2 | 2 | 2 |
| (B') Hydrophobized zinc oxide | 9 | 9 | 9 | 9 | 9 |
| (C) Cyclopentasiloxane | 18 | 18 | 18 | 18 | 18 |
| (C) Isododecane | 8 | 8 | 8 | 8 | 8 |
| (C) Dimethicone | 2 | 2 | 2 | 2 | 2 |
| (C) Isostearic acid | 0.25 | 0.25 | 0.25 | 0.25 | 0.25 |
| (C) Isopropyl myristate | 5 | 5 | 5 | 5 | 5 |
| (C) Diisopropyl sebacate | 1 | 1 | 1 | 1 | 1 |
| (C) PPG-17 | 1 | 1 | 1 | 1 | 1 |
| (D) Ethanol | 10 | 10 | 10 | 10 | 10 |
| (D) Glycerin | 1 | 1 | 1 | 1 | 1 |
| (D) Ion-exchanged water | Balanc e | Balanc e | Balanc e | Balanc e | Balanc e |
| (E) Sorbitan sesquiisostearate | 0.5 | 0.5 | 0.5 | 0.5 | 0.5 |
| (E) PEG-9 polydimethylsiloxyethyl dimethicone | 0.6 | 0.6 | 0.6 | 0.6 | 0.6 |
| (F) Disteardimonium hectorite | 0.5 | 0.5 | 0.5 | 0.5 | 0.5 |
| (F) Tocopherol | 0.05 | 0.05 | 0.05 | 0.05 | 0.05 |
| (F) Dibutylhydroxytoluene | 0.05 | 0.05 | 0.05 | 0.05 | 0.05 |
| (F) EDTA-3Na | 0.1 | 0.1 | 0.1 | 0.1 | 0.1 |
| Total | 100 | 100 | 100 | 100 | 100 |
| Integrated value of absorbance | 48.3 | 42.9 | 41.2 | 45.5 | 45.4 |
| Rate of increase in integrated valu e of absorbance (%) | 93 | 117 | 112 | 88 | 87 |

The emulsion-type cosmetic composition of the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

Some or all of the foregoing embodiments may be described as in the following additional items, although not limited thereto. The various additional items may be employed in combination with the claim(s) in the Scope of Claims.

### {Additional Item 1}

A method of improving UV protection effects, the method comprising:
preparing an emulsion-type cosmetic composition containing an oil-soluble UV absorber and a polymer powder; and
applying the emulsion-type cosmetic composition to the skin, wherein
an average IOB value of a monomer or monomers constituting the polymer powder is from 1 to 10.

### {Additional Item 2}

The method as described in the Additional Item, wherein the emulsion-type cosmetic composition contains:
from 3 to 50% by mass of the oil-soluble UV absorber; and
from 0.2 to 20% by mass of the polymer powder.

### {Additional Item 3}

The method according to the Additional Item, wherein the polymer powder contains a polybutylene succinate powder and/or a polylactic acid powder.

### {Additional Item 4}

An emulsion-type cosmetic composition comprising:
from 3 to 50% by mass of an oil-soluble UV absorber; and
from 0.2 to 20% by mass of a powder containing a polymer powder, wherein
the polymer powder contains a polybutylene succinate powder and/or a polylactic acid powder..

### {Additional Item 5}

The method and/or composition according to the Additional Item,
wherein the polymer powder has an average particle size of from 0.5 to 20 µm.

### {Additional Item 6}

The method and/or composition according to the Additional Item,
wherein the polymer powder has a squalane oil absorption value of 45 mL/100 g or greater.

### {Additional Item 7}

The method and/or composition according to the Additional Item,
wherein the oil-soluble UV absorber contains at least one selected from the group consisting of homosalate, ethylhexyl salicylate, octocrylene, bis-ethylhexyloxyphenol methoxyphenyl triazine, and diethylamino hydroxybenzoyl hexyl benzoate.

### {Additional Item 8}

The method and/or composition according to the Additional Item, wherein the content by percentage of silicone powder in the emulsion-type cosmetic composition is 2% by mass or less with respect to the mass of the emulsion-type cosmetic composition.

### Industrial Applicability

The emulsion-type cosmetic composition of the present disclosure is applicable to cosmetics, cleansers, etc., that are to be applied to the skin. Particularly, the emulsion-type cosmetic composition of the present disclosure is suitably applicable to sun-block cosmetic products.

## Claims

1. An emulsion-type cosmetic composition comprising:
from 3 to 50% by mass of an oil-soluble UV absorber; and
from 0.2 to 20% by mass of a powder containing a polymer powder, wherein
an average IOB value of a monomer or monomers constituting the polymer powder is from 1 to 10.

2. The composition according to claim 1, wherein the polymer powder has an average particle size of from 0.5 to 20 µm.

3. The composition according to claim 1 or 2, wherein the polymer powder has a squalane oil absorption value of 45 mL/100 g or greater.

4. The composition according to any one of claims 1 to 3, wherein the polymer powder contains a polybutylene succinate powder and/or a polylactic acid powder.

5. The composition according to any one of claims 1 to 4, wherein the oil-soluble UV absorber contains at least one selected from the group consisting of homosalate, ethylhexyl salicylate, octocrylene, bis-ethylhexyloxyphenol methoxyphenyl triazine, and diethylamino hydroxybenzoyl hexyl benzoate.
